# EUROPEAN PATENT APPLICATION

(11) **EP 1 629 776 A1**
(43) Date of publication of application: **01.03.2006**
(21) Application number: 05013233.1
(22) Date of filing: 20.06.2005
(51) Int. Cl.: A61B 8/00

(54) **System and method for providing assistance in ultrasound equipment**

(30) Priority: 24.08.2004 KR 2004066786
(71) Applicant: MEDISON CO., LTD., Kangwon-do 250-870 (KR)
(72) Inventor: Yoon, Seung II, Jongno-gu Seoul 110-812 (KR)
(74) Representative: Lorenz, Werner

(57) **Abstract**

The present invention relates to a system and a method for dynamically providing help information associated with the functions of ultrasound equipment. A system for providing help information in a medical ultrasound equipment comprises: a storage unit for storing help information associated with a plurality of functions of the medical ultrasound equipment; an input unit for inputting a plurality of user operation instructions, each of the user operation instructions being associated with at least one of the functions of the ultrasound equipment; an output unit for outputting the help information; and a control unit responsive to a user's input of one of the user operation instructions for controlling a particular help information associated with a particular function of the ultrasound equipment, which in turn is associated with the inputted user operation instruction, to be outputted through the output unit.

## Description

The present invention generally relates to ultrasound equipment, and more particularly to a system and a method for dynamically providing assistance associated with functions of the ultrasound equipment to a user.

A user of ultrasound equipment often desires to be provided with information associated with various functions of the ultrasound equipment in order to acquire knowledge of how to operate the relevant functions. To this end, the user may either refer to an operation manual or search for an explanation of a relevant function by using a help function provided on the ultrasound equipment itself. However, the user who is unfamiliar with the functions of the ultrasound equipment may feel it inconvenient to refer to a manual every time a need arises for the user to operate some of the functions of the equipment.

A conventional help function used in the ultrasound equipment provides information if the user activates a help window by using a key (or a shortcut key) placed on a key panel in the ultrasound equipment. The user can then search for desired information on the help window.

However, to properly operate the conventional help function, the user is required to repeat the search process every time when a need arises to acquire information on the functions of the equipment. In such a case, the user is confronted with a difficulty in that he/she must input a proper keyword so as to search for the desired information. Also, there is a disadvantage in that it takes a long time to search for the desired information. Furthermore, since a screen region where information is displayed is limited, the help window has to be activated whenever the user requires the information associated with the function, in which the help window must then be closed again for the operation of the ultrasound image equipment. Thus, a process for providing the information associated with the functions of the ultrasound equipment becomes very complicated.

Further, in the operation of the ultrasound equipment, since the text provided on the key panel of the ultrasound equipment is not in a descriptive language but in a summarized format, there is a problem in that it is difficult for the user to understand the meaning of the function represented by each key.

It is an objective of the present invention to provide to a system and a method for dynamically providing help information associated with the functions of ultrasound equipment by using a help mode.

In accordance with an aspect of the present invention, there is provided a system for providing help information in medical ultrasound equipment, which comprises: a storage unit for storing help information associated with a plurality of functions of the medical ultrasound equipment; an input unit for inputting a plurality of user operation instructions, each of the user operation instructions being associated with at least one of the functions of the ultrasound equipment; an output unit for outputting the help information; and a control unit responsive to a user's input of one of the user operation instructions for controlling a particular help information associated with a particular function of the ultrasound equipment, which in turn is associated with the inputted user operation instruction, to be outputted through the output unit.

In accordance with another aspect of the present invention, there is provided a method for providing help information in medical ultrasound equipment, which comprises the following steps: a) storing help information associated with a plurality of functions of the medical ultrasound equipment; b) inputting a user operation instruction for operating one of the functions of the ultrasound equipment; and c) controlling a particular help information to be outputted in response to the inputted operation instruction.

The above and other objects and features of the present invention will become apparent from the following description of preferred embodiments provided in conjunction with the accompanying drawings, in which:
Fig. 1 is a block diagram showing a system for providing help information in ultrasound equipment in accordance with the present invention; and
Fig. 2 is a flow chart showing a method for providing help information by using a help mode in ultrasound equipment in accordance with the present invention.

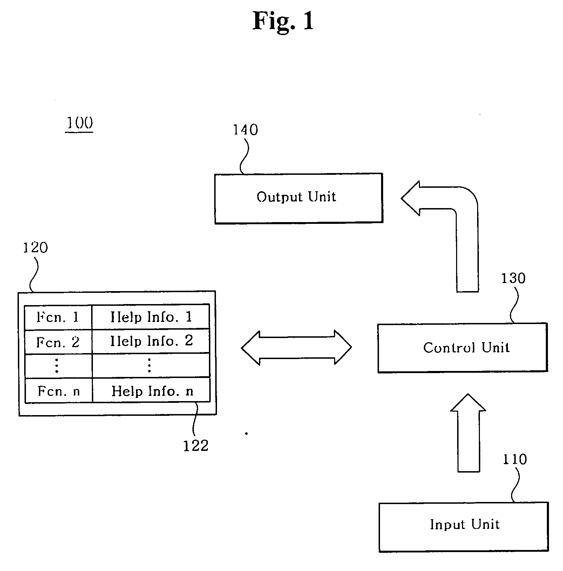

Fig. 1 is a block diagram showing a system for providing help information in medical ultrasound equipment in accordance with the present invention.

Referring to Fig. 1, the system 100 includes an input unit 110, a storage unit 120, a control unit 130 and an output unit 140.

The input unit 110 receives the user's operation instructions associated with a help mode selection and functions of the ultrasound equipment. The input unit 110 may be a keyboard, a touch screen and/or a pointing device like a mouse. An additional key (e.g., a help mode selection key) may be provided to select the help mode. Also, the help mode selection can be carried out by using a combination of keys assigned to other functions of the ultrasound equipment. The help mode may be selected before operating the function of the ultrasound equipment or while operating the function of the ultrasound equipment.

The storage unit 120 stores help information associated with each function of the ultrasound equipment. The help information represents detailed information associated with the functions to be selected by the user. The help information associated with the functions may be stored in a memory, a hard disk or a database in a table format 122.

When the user chooses one of the functions of the ultrasound equipment through the input unit 110, the control unit 130 checks whether the user selects the help mode. If it is determined that the user selects the help mode, the control unit 130 searches for suitable help information associated with the chosen function in the storage unit 120. Thereafter, the control unit 130 reads out the help information from the storage unit 120, which is associated with the function chosen by the user. The control unit 130 outputs the chosen help information through the output unit 140. The output unit 140 may be a display device such as a CRT (Cathode Ray Tube) or LCD (Liquid Crystal Display) displaying the help information with text or graphics, or a speaker outputting the help information through sound.

Also, the control unit 130 receives the help information through the input unit 110 from the user for updating the help information associated with the functions of the ultrasound equipment, which are stored in the storage unit 120.

On the other hand, if it is determined that the help mode is not selected, then the control unit 130 directly operates the function in the ultrasound equipment, which is chosen by the user, without searching for the help information stored in the storage unit 120.

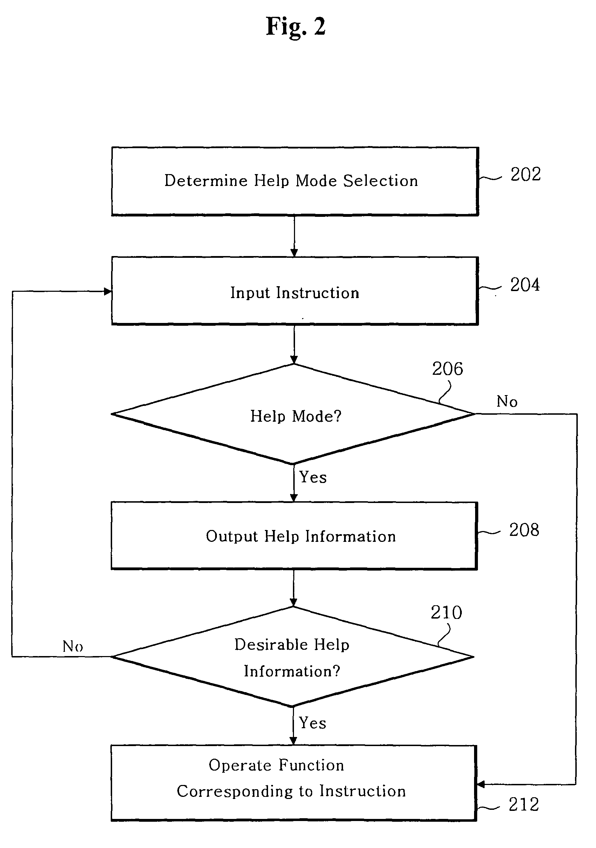

A method for providing the help information associated with the functions of the ultrasound equipment to the user in response to a selection of the help mode will be described below in view of Fig. 2.

Fig. 2 is a flow chart showing a method for providing the help information in response to a selection of the help mode in accordance with the present invention.

Referring to Fig. 2, the user determines whether to select the help mode before operating the ultrasound equipment or at the time of operating the ultrasound equipment at step 202. The help information associated with the functions of the ultrasound equipment is set at the initial setup stage of the ultrasound equipment and may be updated by the user when the user operates the ultrasound equipment. The help information is stored in the storage unit 120 for reference by the control unit 130.

When the user inputs a certain operation instruction for choosing one of the functions of the ultrasound equipment through the input unit 110 at step 204, the control unit 130 checks whether the help mode is selected at step 206. If it is determined that the user selected the help mode, the help information associated with the corresponding function is outputted through the output unit 140 before the corresponding function of the ultrasound equipment actually operates at step 208.

On the other hand, if the user does not wish to confirm the help information associated with the function corresponding to the operation instruction because the user is familiar with the function of the ultrasound equipment, the user does not have to select the help mode. That is, when the help mode is not selected, the corresponding function of the ultrasound equipment is directly executed in response to the operation instruction without the help information associated with the corresponding function being outputted through the output unit 140 at step 212.

If the help mode is selected, it is determined whether the help information, which is outputted through the output unit 140 in response to the user's operation instruction, is associated with the function of the ultrasound equipment intended by the user at step 210. In case that the outputted help information is associated with the function of the ultrasound equipment intended by the user, the current function of the ultrasound equipment is executed at step 212.

On the other hand, in case that the outputted help information is independent from the information associated with the function intended by the user, the current function of the ultrasound equipment is canceled and the process returns to step 204 for inputting another operation instruction.

As mentioned above, the help information can be provided to the user who is unfamiliar with the ultrasound equipment and may be not provided to the user who is familiar with the ultrasound equipment in accordance with the present invention. That is, the help information associated with the functions of the ultrasound equipment can be dynamically provided by selectively setting the help mode in accordance with the present invention.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. An system for providing help information in a medical ultrasound equipment, comprising:
a storage unit for storing help information associated with a plurality of functions of the medical ultrasound equipment;
an input unit for inputting a plurality of user operation instructions, each of the user operation instructions being associated with at least one of the functions of the ultrasound equipment;
an output unit for outputting the help information; and
a control unit responsive to a user's input of one of the user operation instructions for controlling a particular help information associated with a particular function of the ultrasound equipment, which in turn is associated with the inputted user operation instruction, to be outputted through the output unit.

2. The system as recited in Claim 1, wherein the input unit includes a help mode selection key for selecting a help mode.

3. The system as recited in Claim 1, wherein the output unit is a display device for providing the help information with text or graphics or a speaker for providing the help information with sound.

4. The system as recited in Claim 1, wherein the help information is stored at an initial setup stage of the ultrasound equipment.

5. The system as recited in Claim 4, wherein the help information is updated by inputting help information at the time of operating the ultrasound equipment through the input unit.

6. A method for providing help information in a medical ultrasound equipment, comprising the steps of:
a) storing the help information associated with a plurality of functions of the medical ultrasound equipment;
b) inputting a user operation instruction for operating one of the functions of the ultrasound equipment; and
c) controlling a particular help information to be outputted in response to the inputted operation instruction.

7. The method as recited in Claim 6, wherein step c) includes the steps of:
c-1) checking whether a help mode is selected in response to the inputted operation instruction;
c-2) searching for the particular help information when the help mode is selected; and
c-3) reading out the particular help information.

8. The method as recited in Claim 7, further comprising the step of updating the help information by inputting the help information.

9. The method as recited in Claim 6, wherein step c) further comprises the step of c-4) outputting the particular help information through a display device or a speaker.

10. The method as recited in Claim 6, further comprising the steps of:
d) checking whether the help information corresponds to the function of the ultrasound equipment intended by a user; and
e) if the help information is independent from the function of the ultrasound equipment intended by the user, inputting another user operation instruction.
